(19) Europäisches Patentamt
European Patent Office
Office européen des brevets

(11) Veröffentlichungsnummer: **0 028 757**
**A1**

# (12) EUROPÄISCHE PATENTANMELDUNG

(21) Anmeldenummer: **80106577.2**

(22) Anmeldetag: **25.10.80**

(51) Int. Cl.³: **C 07 D 233/58**
**A 61 K 31/415**

(30) Priorität: **06.11.79 DE 2944662**

(43) Veröffentlichungstag der Anmeldung:
**20.05.81 Patentblatt 81/20**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Lieb, Folker, Dr.**
**Alfred-Kubin-Strasse 1**
**D-5090 Leverkusen(DE)**

(72) Erfinder: **Oediger, Hermann, Dr.**
**Roggendorfstrasse 51**
**D-5000 Koeln 80(DE)**

(72) Erfinder: **Busse, Wolf-Dieter, Dr.**
**Pahlkestrasse 65**
**D-5600 Wuppertal 1(DE)**

(72) Erfinder: **Seuter, Friedel, Dr.**
**Moospfad 16**
**D-5600 Wuppertal 1(DE)**

(72) Erfinder: **Perzborn, Elisabeth, Dr.**
**Sadowastrasse 65**
**D-5600 Wuppertal 1(DE)**

## (54) Cycloheptatrienylimidazolderivate, Verfahren zu ihrer Herstellung sowie ihre Verwendung in Arzneimitteln.

(57) Cycloheptatrienylimidazolderivate der allgemeinen Formel

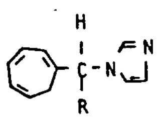

in welcher
R für Wasserstoff, gegebenenfalls substituiertes Alkyl oder gegebenenfalls substituiertes Phenyl steht,
als solche oder in Form ihrer physiologisch unbedenklichen Salze, eine Verfahren zu ihrer Herstellung sowie ihre Verwendung in Arzneimitteln, insbesondere in Mitteln mit antithromboembolischer Wirkung.

EP 0 028 757 A1

0028757

BAYER AKTIENGESELLSCHAFT 5090 Leverkusen, Bayerwerk
Zentralbereich
Patente, Marken und Lizenzen KS/kl-c
Ia (Pha)

Cycloheptatrienylimidazolderivate, Verfahren zu ihrer Herstellung sowie ihre Verwendung in Arzneimitteln

Die vorliegende Erfindung betrifft neue Cycloheptatrienylimidazolderivate, ein Verfahren zu ihrer Herstellung sowie ihre Verwendung in Arzneimitteln, insbesondere in Mitteln mit antithromoboembolischer Wirkung.

Thrombose und arteriosklerotische Gefäßwandveränderungen werden vor allem durch die Wechselwirkung zweier Metaboliten der Arachidonsäure, nämlich durch das Thromboxan $A_2$ ($TXA_2$) auf der einen und durch das Prostacyclin ($PGI_2$) auf der anderen Seite gesteuert. $TXA_2$ wirkt auf die Blutplättchen aggregierend und $PGI_2$ hat eine antiaggregierende Wirkung. Darüber hinaus wirkt $TXA_2$ vasokonstriktorisch und $PGI_2$ vasodilatatorisch.

Bei einer Reihe von thrombo-embolischen und ischämischen Erkrankungen führt Hyperaggrebilität der Plättchen bzw. ein erhöhter Plättchenverbrauch zu einer gesteigerten Thromboxansynthese, so daß das $TXA_2$- und $PGI_2$-Gleichgewicht gestört ist. Es ist deshalb zur

Therapie und Prophylaxe von thrombo-embolischen und ischämischen Erkrankungen wünschenswert, die Thromboxansynthese zu hemmen und damit die protektive Eigenschaft des $PGI_2$ zu erhöhen.

Aus der Europäischen Patentanmeldung Nr. 951 sind bereits Imidazolderivate bekannt, die eine antithromboembolische Wirkung besitzen. Cycloheptatrienylimidazole sind jedoch bisher noch nicht namentlich beschrieben. Aufgrund ihrer überraschend vorteilhaften thromboxansynthesehemmenden Wirkung stellen sie eine erfinderische Auswahl aus der Vielzahl der bisher bekannten Imidazolderivate dar.

Die Erfindung betrifft neue Cycloheptatrienylimidazolderivate der allgemeinen Formel (I)

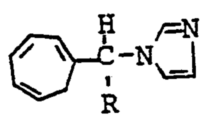

(I)

in welcher

R für Wasserstoff, geradkettiges oder verzweigtes Alkyl, welches gegebenenfalls durch Halogen oder Trifluormethyl substituiert ist, oder für Phenyl, welches gegebenenfalls durch Halogen, Alkyl oder Trifluormethyl substituiert ist, steht,

als solche oder in Form ihrer physiologisch unbedenklichen Salze.

Die Herstellung der Verbindungen der allgemeinen Formel (I) erfolgt beispielsweise, indem man Imidazole der allgemeinen Formel (II)

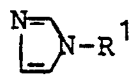

(II)

in welcher

$R^1$ für Wasserstoff oder ein Alkalimetall steht,

mit einem Cycloheptatrienderivat der allgemeinen Formel (III)

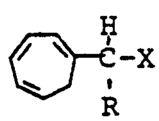

(III)

in welcher

R die oben angegebene Bedeutung hat und

X für Halogen oder einen Alkyl- oder Arylsulfonylsäurerest steht, der gegebenenfalls durch niederes Alkyl, Halogen oder Nitro substituiert ist,

gegebenenfalls in Gegenwart von inerten organischen Lösungsmitteln bei Temperaturen zwischen 0 und 150°C umsetzt.

Verwendet man als Ausgangsstoffe Imidazolylnatrium und 1-Chlormethylcyclohepta-1,3,5-trien, so kann der Reaktionsablauf durch folgendes Formelschema wiedergegeben werden:

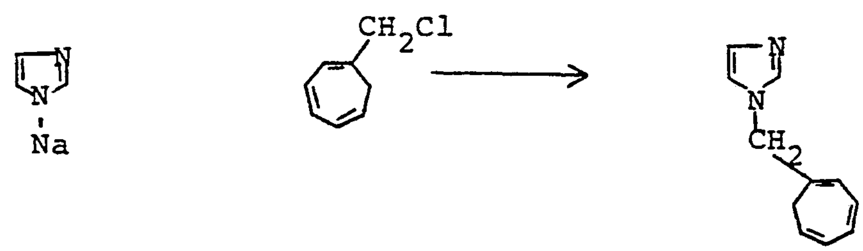

Die als Ausgangsstoffe verwendeten Imidazolderivate der allgemeinen Formel (II) sind bekannt (vgl. Beilstein, 23 (2), 35).

Die Imidazolylmetallverbindungen der Formel (II) können direkt eingesetzt werden oder unmittelbar bei der Reaktion in inerten Lösungsmitteln aus Imidazol und den entsprechenden Alkalimetallverbindungen hergestellt werden. Als Alkalimetallverbindungen seien vorzugsweise genannt Natriumhydroxid, Kaliumhydroxid, Alkalialkoholate wie Natriummethylat, Kalium-tert.-butylat, Alkalihydride wie Natriumhydrid und Kaliumhydrid, metallorganische Verbindungen wie Butyllithium, tert.-Butyllithium und Phenyllithium.

Die als Ausgangsstoffe verwendeten Cycloheptatriene

der allgemeinen Formel (III) sind ebenfalls bekannt oder können nach bekannten Methoden hergestellt werden (vgl. T. Asao et al., Chemistry Letters, Seite 41 (1978) und J.A. Blair et al., Journal Chemical Society, Seite 1592 (1971) und DT-OS 28 41 790).

Von besonderem Interesse sind Verbindungen der allgemeinen Formel (III) in welcher

X vorzugsweise für Chlor, Brom oder Iod, für einen Alkylsulfonsäurerest mit 1 bis 4 Kohlenstoffatomen, insbesondere für den Methan- und Ethansulfonsäurerest oder für einen Arylsulfonsäurerest, vorzugsweise für den Phenylsulfonsäurerest, der gegebenenfalls durch Alkyl mit 1 oder 2 Kohlenstoffatomen oder durch Chlor oder Brom oder durch Nitro substituiert ist, insbesondere für den p-Toluolsulfonsäurerest steht.

In den allgemeinen Formeln (I) und (III) steht

R vorzugsweise für Wasserstoff, geradkettiges oder verzweigtes Alkyl mit 1 bis 6 Kohlenstoffatomen, gegebenenfalls substituiert durch Halogen oder durch den Trifluormethylrest, für Phenyl, gegebenenfalls substituiert durch Halogen, insbesondere Fluor oder Chlor, Trifluormethyl oder Alkyl mit 1 bis 2 Kohlenstoffatomen.

Als Wirkstoffe seien im einzelnen beispielhaft genannt:
N-[1-(Cyclohepta-1,3,5-trien-1-yl)-propyl]-imidazol
N-[1-(Cyclohepta-1,3,5-trien-1-yl)-benzyl]-imidazol
N-[1-(Cyclohepta-1,3,5-trien-1-yl)-p-chlorbenzyl]-imidazol.

Besonders hervorgehoben seien Verbindungen, in denen R Wasserstoff, Methyl, Ethyl oder Phenyl bedeutet.

Als Verdünnungsmittel kommen bei der Durchführung des erfindungsgemäßen Verfahren alle inerten organischen Lösungsmittel in Frage. Hierzu gehören vorzugsweise cyclische oder acyclische Ether wie Tetrahydofuran, Dioxan oder Dimethoxymethan, aliphatische Carbonsäurenitrile mit 2 bis 6 Kohlenstoffatomen wie Acetonitril oder Propionitril, aliphatische N-substituierte Carbonsäureamide, insbesondere Dimethylformamid oder Dimethylacetamid, Phosphorsäureamide wie Hexamethylphosphorsäuretriamid und Sulfoxide wie Dimethylsulfoxid.

Die Reaktionstemperaturen können in einem größeren Bereich variiert werden. Im allgemeinen arbeitet man zwischen 0 und 150°C, vorzugsweise zwischen 10 und 100°C.

Bei der Durchführung des erfindungsgemäßen Verfahrens setzt man 1 Mol der Verbindung (III) mit 2 bis 6 Mol, vorzugsweise mit 2 bis 4 Mol Imidazol, oder bei Verwendung von Imidazoliden mit 0,9 bis 1,1 Mol, vorzugsweise mit 0,95 bis 1,0 Mol Imidazolid um.

Die Reaktionsdauer ist von der Temperatur abhängig und liegt zwischen 1 und 10 Stunden.

Die Reinigung der erfindungsgemäßen Verbindungen der allgemeinen Formel (I) erfolgt nach bekannten Methoden entweder durch Umkristallisation, Destillation oder durch chromatographische Methoden.

Überraschenderweise hemmen die erfindungsgemäßen Verbindungen der allgemeinen Formel (I) die Thromboxansynthese stärker und spezifischer als die aus dem Stand der Technik bekannten Imidazolderivate. Aufgrund ihrer unerwartet starken und spezifischen Wirkung, die mit guter Verträglichkeit gekoppelt ist, stellen die erfindungsgemäßen Verbindungen eine Bereicherung der Pharmazie und eine erfinderische Auswahl aus dem Stand der Technik dar.

Die vorliegende Erfindung betrifft ebenfalls galenische Zubereitungen der erfindungsgemäßen Verbindungen in Form von Pillen, Tabletten, Dragees, Cremes, Suppositorien, Emulsionen, Suspensionen sowie Infusions- und Injektionslösungen.

Diese Zubereitungsformen können je nach Bedarf lokal, parenteral oder oral verabreicht werden.

Besonders geeignet sind Formulierungen, die etwa 0,1 bis 10 Gew.-% an Wirkstoff enthalten. Besonders bevorzugt sind wäßrige Lösungen, insbesondere gepufferte Lösungen mit einem pH von 6 bis 8.

Die Herstellung der galenischen Zubereitungsformen erfolgt nach üblichen Methoden gegebenenfalls unter Verwendung bekannter Hilfs- und Trägerstoffe. Die Dosierung erfolgt vorzugsweise in einem Bereich von 0,05 bis 100 mg/kg Körpergewicht, insbesondere von 0,1 bis 50 mg/kg Körpergewicht.

Ausführungsbeispiele

Beispiel 1

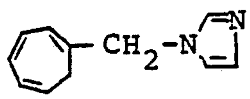

N-[(Cyclohepta-1,3,5-trien-1-yl)-methyl]-imidazol

Zu 14 g Imidazol in 200 ml absolutem Dimethylformamid gibt man portionsweise 6 g Natriumhydrid (80 %ig). Man rührt bis zum Ende der Gasentwicklung bei einer Temperatur, die unter 20°C liegt. Darauf läßt man 28 g Chlormethyl-cyclohepta-1,3,5-trien in 100 ml absolutem Dimethylformamid einfließen und erhitzt 3 Stunden auf 60°C. Das Dimethylformamid wird abdestilliert, der Rückstand in 1000 ml Essigester aufgenommen. Vom Unlöslichen filtriert man ab und dampft das Filtrat im Wasserstrahlvakuum ein. Man erhält 32 g Rückstand, der an Kieselgel mit Methylenchlorid/10 % Methanol chromatographiert 25 g N-[(Cyclohepta-1,3,5-trienyl)-methyl]-imidazol (Ausbeute: 73 %) ergibt.

Kp 114 - 119°C/0,07 mbar.

$^1$H-NMR ($CDCl_3$): $\delta$ = 2,20 (d, H = 7 Hz, 2H), 4,63 (bs, 2H) und 5,24 und 5,39 ppm (d von t, J = 8 und 10 Hz, 1H).

Beispiel 2

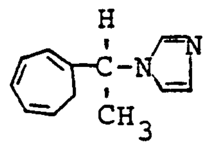

N-[1-(Cyclohepta-1,3,5-trien-1-yl)-ethyl]-imidazol

Analog Beispiel 1 erhält man aus 1-Chlorethylcyclohepta-1,3,5-trien N-[1-(Cyclohepta-1,3,5-trien-1-yl)-ethyl]-imidazol in einer Ausbeute von 57 %.

IR (Film): $\nu$ = 1640, 1510 und 1439 $cm^{-1}$.

Patentansprüche

1. Cycloheptatrienylimidazolderivate der allgemeinen Formel (I)

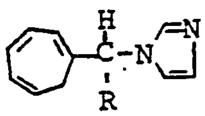

(I)

in welcher

R für Wasserstoff, gegebenenfalls substituiertes Alkyl oder gegebenenfalls substituiertes Phenyl steht,

als solche oder in Form ihrer physiologisch unbedenklichen Salze.

2. Verbindungen der allgemeinen Formel (I) gemäß Anspruch 1 in welcher

R für Wasserstoff, Alkyl mit 1 bis 4 Kohlenstoffatomen, gegebenenfalls durch Halogen oder Trifluormethyl substituiert oder für Phenyl, gegebenenfalls durch Fluor, Chlor, Trifluormethyl oder Alkyl mit 1 bis 2 Kohlenstoffatomen substituiert, steht.

3. Verbindungen der allgemeinen Formel (I) gemäß

Anspruch 1 in welcher

R für Wasserstoff oder Alkyl mit 1 bis 4 Kohlenstoffatomen oder für Phenyl steht.

4. Verfahren zur Herstellung von Verbindungen der allgemeinen Formel (I) gemäß Anspruch 1, dadurch gekennzeichnet, daß man Imidazole der allgemeinen Formel (II)

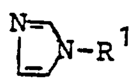

(II)

in welcher

$R^1$ für Wasserstoff oder ein Alkalimetall steht,

mit einem Cycloheptatrienderivat der allgemeinen Formel (III)

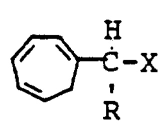

(III)

in welcher

R die oben angegebene Bedeutung hat und

X für Halogen oder einen Alkyl- oder Arylsulfonylsäurerest steht, der gegebenenfalls durch niederes Alkyl, Halogen oder Nitro substituiert ist,

gegebenenfalls in Gegenwart von inerten organischen Lösungsmitteln bei Temperaturen zwischen 0 und 150°C umsetzt.

5. Arzneimittel, enthaltend mindestens eine Verbindung gemäß Anspruch 1.

6. Antithromboembolisch wirksame Arzneimitteln, enthalend mindestens eine Verbindung gemäß Anspruch 1.

7. Verfahren zur Herstellung von Arzneimitteln, dadurch gekennzeichnet, daß man Verbindungen gemäß Anspruch 1, gegebenenfalls unter Verwendung von üblichen Hilfs- und Trägerstoff, in eine geeignete Applikationsform überführt.

8. Verwendung von Verbindungen gemäß Anspruch 1 in antithromboembolisch wirksamen Arzneimitteln.

0028757

Europäisches Patentamt

# EUROPÄISCHER RECHERCHENBERICHT

Nummer der Anmeldung

EP 80 10 6577.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | EP - A1 - 0 000 951 (WELLCOME FOUNDATION LTD.)<br>* Ansprüche 1, 11, 18; Seite 4, Zeilen 5 bis 10 *<br>-- | 1,5-7 |
| | EP - A1 - 0 000 950 (WELLCOME FOUNDATION LTD.)<br>* Ansprüche 1, 7; Seite 3, Zeilen 12 bis 16 *<br>-- | 1,4 |
| P | DE - A1 - 2 855 329 (GRÜNENTHAL GMBH)<br>* Ansprüche 1, 10, 13 *<br>-- | 1,4,5 |
| P | EP - A1 - 0 015 002 (WELLCOME FOUNDATION LTD.)<br>* Ansprüche 10, 11, 14 *<br>-- | 4,5-7 |
| | GB - A - 1 364 312 (BEECHAM GROUP LTD.)<br>* Seite 1, Spalte 2, Zeilen 54 bis 57 *<br>---- | 1 |

KLASSIFIKATION DER ANMELDUNG (Int. Cl.³)

C 07 D 233/58
A 61 K 31/415

RECHERCHIERTE SACHGEBIETE (Int. Cl.³)

A 61 K 31/415
C 07 D 233/58

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 16-02-1981 | FROELICH |